# EUROPEAN PATENT APPLICATION

(11) **EP 1 250 920 A1**
(43) Date of publication of application: **23.10.2002**
(21) Application number: 02252512.5
(22) Date of filing: 08.04.2002
(51) Int. Cl.: A61K 9/14, A61K 31/195, A61K 31/785

(54) **Unloaded ion exchange resins for the extended release of active ingredients**

(30) Priority: 09.04.2001 US 282442 P
(71) Applicant: ROHM AND HAAS COMPANY, Philadelphia, Pennsylvania 19106-2399 (US)
(72) Inventor: Hughes, Lyn, Harleysville, Pennsylvania 19438 (US); Bellamy, Simon Andrew, Redhill, Surrey, RH1 6DD (GB); Hann, Christina, Gwynedd, Pennsylvania 19436 (US)
(74) Representative: Kent, Venetia Katherine

(57) **Abstract**

A dosage form is described that gives an extended release of active ingredients using unloaded ion exchange resins, that does not require the manufacture of a resinate.

## Description

### BACKGROUND OF THE INVENTION

The concept of controlled, extended, or modified release of biologically active ingredients is well known, and can be very advantageous in the administering of said active ingredients. For example, in the area of pharmaceuticals, by extending the release of a pharmaceutically active ingredient it is possible to increase the time during which the blood plasma concentration of said active ingredient is between an upper limit, defined by the toxicological properties of said active ingredient and a lower limit defined by the efficacy of the said active ingredient. Additionally, in the area of water treatment chemicals, controlled release can result in the more efficient use of the active ingredient because similar limits exist where the upper limit is defined by processing requirements, such as limiting corrosion and reducing toxicity to non-targeted organisms, and the lower limit is defined by efficacy. Further in the area of agricultural chemicals, controlled release is beneficial because similar limits exist where the upper limit is defined by pollution of the environment, and toxicity to non-targeted organisms, and the lower limit is defined by efficacy.

There are many examples of different methods known in the industry for modifying the release rate of active ingredients, including many commercialized formulations. One of the methods that has been used in the pharmaceutical art is to convert the drug into a complex with an ion exchange resin to form a resinate. Resinates are salts formed between ion exchange resins and ionizable active ingredients. Cation exchange resins form resinates with basic active ingredients. Anion exchange resins form resinates with acidic active ingredients. In the resinate the active ingredient and the resin are in their ionized forms. When resinates are exposed to fluids such as physiological fluids the active ingredient can be released from the resinate by the mechanism of ion exchange. The rate of release of active ingredients from resinates depends on several factors which are well known in the industry. These include, but are not limited to, degree of cross-linking of the ion exchange resin, the particle size of the resinate, the pK of the functional groups of the resin, the solubility of the active ingredient in the release fluid, the ionic strength and pH of the release fluid, the pK of the active ingredient, the molecular weight of the active ingredient, and the temperature. Coating the resin with a permeable membrane can also change the rate of release. Coating the resin with non-permeable membranes can change the conditions under which the release takes place depending on the conditions under which the membrane dissolves.

Using the variables described above, the resinate can be used to provide control of the release rate, providing an extended release of the active ingredient. While this method of controlling the release rate is effective it is necessary to manufacture and characterize said resinate. The manufacture of the resinate contributes significant cost to the overall cost of manufacturing the final dosage form. Costs are derived from processing equipment, processing time, labor, analysis and packing . For example, in US 4,510,128, a process is described for the manufacture of a resinate comprising diclofenac sodium and cholestyramine for the purpose of extended release. In Example 1 of US 4,510,128 the manufacturing process involves treatment with sodium hydroxide, hydrochloric acid, multiple washings with water and isopropanol, drying, and finally a 12 hour drug loading step. Total processing time can be estimated to be approximately 40 hours. Characterization of the resinate can also be problematic because of the limited number of analytical methods suitable for solids that are insoluble. The resinate manufacture and characterization of the resinate must also be reviewed by regulatory agencies such the US Food and Drug Administration. This can add both time and cost to the development of a formulation. While the benefits of using a resinate to control the release rate profile of active ingredients frequently justify such costs, it is clear that it would very advantageous to avoid the necessity of making a resinate.

Sriwongjanya and Bodmeier (Eur J Pharm Biopharm, volume 46, pages 321-327, 1998) teaches the use of gellular matrices, such as hydroxypropylmethyl cellulose, to create a microenvironment within a dosage form that facilitates the loading of either propranolol or diclofenac sodium onto ion exchange resins after administration. In this device the loading takes place within the swellable gel matrix.

Applicants have surprisingly found that the benefits of using a resinate can be achieved without manufacturing a resinate or using a swellable matrix. Specifically, in the present invention an unloaded resin (b.) is administered at the same time as an ionizable active ingredient (a.). The combination of the unloaded resin and ionizable active ingredient creates a release rate profile of the same curved shape as that obtained using a pre-made resinate. By suitable manipulation of the composition the release rate can be made to be the same as using a resinate, thereby removing the need to prepare, isolate and characterize the resinate

The following terms have the following meanings herein:

The term "release rate profile", as used herein, means the rate at which the active ingredient that is loaded on the resin appears in solution in the release medium. This can be expressed in terms of the instantaneous concentration of the active ingredient in solution as a function of time, or expressed in terms of the percentage of total active ingredient available that has appeared in solution in the release medium as a function of time.

The term "release medium" and as used herein, means the liquid medium into which the active ingredients is being released. Examples of release media can be water, simulated intestinal fluid, simulated gastric fluid, simulated saliva, or the authentic physiological versions of these fluids, water, and various buffer solutions.

The term "ion exchange resin", as used herein, means any insoluble polymer that can act as an ion exchanger.

The term "release", as used herein, means the transfer of active ingredient from the resinate into the release medium. When applied to a resin or resinate, the term "absorption", as used herein, means the reverse of release, namely the transfer of active ingredient from the medium into the ion exchange resin or resinate.

The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)

The term "resinate," as used herein, means a complex formed between an active ingredient and an ion exchange resin. It is also known as a loaded resin. The term "resinate" can also be expressed as an active ingredient/ion exchange resin complex.

Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

Ion exchange resins are manufactured in different forms. These forms can include spherical and non-spherical particles with size in the range of 0.00001mm to 2mm. The non-spherical particles are frequently manufactured by grinding of the spherical particles. Products made in this way typically have particle size in the range 0.0001mm to 0.2mm. The spherical particles are frequently known in the art as 'Whole Bead.' The non-spherical particles are frequently known in the art as 'Powders.'

### STATEMENT OF THE INVENTION

The present invention relates to the use of ion exchange resins to control the rate at which active ingredients are released into a release medium. Specifically, the present invention relates to a dosage form comprising:
a. an ionizable active ingredient;
b. an unloaded ion exchange resin.

### DETAILED DESCRIPTION OF THE INVENTION

The present invention relates to a dosage form comprising:
a. an ionizable active ingredient;
b. an unloaded ion exchange resin.

Ion exchange resins useful in the practice of the present invention include, but are not limited to, anionic exchange resins and cationic exchange resins. Preferably, said resins are suitable for human and animal ingestion when the application is pharmaceutical.

Preferred anionic exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g.

More preferred anionic exchange resins include, but are mot limited to, styrenic strongly basic anion exchange resins with a quaternary amine functionality having a weight capacity of 0.1 to 6 meq/g, and styrenic weakly basic anion exchange resins with a tertiary amine functionality having a weight capacity of 0.1 to 8.5 meq/g, acrylic or methacrylic strongly basic anion exchange resins with a quaternary amine functionality having a weight capacity of 0.1 to 8 meq/g, and acrylic or methacrylic weakly basic anion exchange resins with a tertiary amine functionality having a weight capacity of 0.1 to 12 meq/g, and allylic and vinylic weakly basic anion exchange resins with primary, secondary, or tertiary amine functionalities having a weight capacity of 0.1 to 24 meq/g.

Most preferred anionic exchange resins include, but are not limited to, styrenic strongly basic anion exchange resins with quaternary amine functionality with weight capacity of 0.1 to 6 meq/g and acrylic anion exchange resins with tertiary amine functionality with weight capacity of 0.1 to 12 meq/g. Styrenic strongly basic anion exchange resins with quaternary amine functionalities with weight capacities of 4.0 to 4.5 meq/g are also known as cholestyramine resins.

Preferred cationic exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g.

More preferred cationic exchange resins include, but are not limited to, styrenic strongly acidic cation exchange resins with a sulfonic acid functionality having a weight capacity of 0.1 to 8 meq/g; and styrenic weakly acidic cation exchange resins with a phenolic acid functionality having a weight capacity of 0.1 to 8.5 meq/g; and acrylic or methacrylic weakly acidic cation exchange resins with a carboxylic or phenolic acid functionality with a weight capacity of 0.1 to 14 meq/g.

Most preferred cationic exchange resins include, but are not limited to, styrenic strongly acidic cation exchange resin with sulfonic acid functionality with weight capacity of 0.1 to 8 meq/g, and acrylic or methacrylic weakly acidic cation exchange resin with a carboxylic acid functionality with weight capacity of 0.1 to 14 meq/g.

Ion exchange resins useful in this invention have a moisture content between 0% and the water retention capacity of said resin.

Ion exchange resins useful in this invention are in powder or whole bead form.

Strongly acidic and weakly acidic cation exchange resins useful in the practice of the present invention are in the acid form or salt form or partial salt form.

Strongly basic anion exchange resins useful in this invention are in the salt form.

Weakly basic anion exchange resins useful in this invention are in the free-base form or salt form or partial salt form.

The particle size of resins useful in the invention will be defined by the desired release rate profile Typical particle sizes are from 0.00001mm to 2mm. The preferred size is 0.001mm to 1mm. The most preferred size is 0.001mm to 1.0mm

The compositions of the present invention can optionally be coated.

Permeable coatings useful in this invention are well know to one skilled in the art and include Eudragit® RL100, and Eudragit® RS100 (Rohm-Pharma Darmstadt, Germany)

Non-permeable coatings useful in this invention are well known to one skilled in the art and include Aquacoat® CPD (FMC Corporation, Philadelphia, PA, USA), Eudragit® E100, Eudragit® L100, Eudragit® S100 (Rohm-Pharma Darmstadt, Germany), Kollicoat® MA 30 DP (BASF Aktiengesellschaft, Ludwigshafen, Germany).

Active ingredients useful in the practice of the present invention include, but are not limited to, pharmaceutically active ingredients, vitamins, flavors, fragrances, water treatment chemicals such as dispersants, corrosion inhibitors, chelants, biocides, and scale inhibitors, and agricultural chemicals including pesticides, herbicides, fertilizers, and nutrients, that have acidic or basic ionizable groups.

Pharmaceutically active ingredients useful in the practive of this invention are those that include acidic or basic ionizable groups and include, but are not limited to, indomethacin, salicylic acid, ibuprofen, sulindac, diclofenac, piroxicam, naproxen, timolol, pilocarpine, acetylcholine, dibucaine, thorazine, promazine, chlorpromazine, acepromazine, aminopromazine, perazine, prochlorperazine, trifluoroperazine, thioproperazine, reserpine, deserpine, chlorprothixene, tiotixene, haloperidol, moperone, trifluorperidol, timiperone, droperidol, pimozide, sulpiride, tiapride, hydroxyzine, chlordiazepoxide, diazepam, propanolol, metoprolol, pindolol, imipramine, amitryptyline, mianserine, phenelzine, iproniazid, amphetamines, dexamphetamines, fenproporex, phentermine, amfepramone, pemoline, clofenciclan, cyprodenate, aminorex, mazindol, progabide, codergoctine, dihydroergocristine, vincamone, citicoline, physostigmine, pyritinol, meclofenoxate, lansoprazole, nifedipine, risperidone, clarithromycin, cisapride, nelfinavir, midazolam, lorazepam, nicotine, prozac, erythromycin, ciprofloxacin, quinapril, isotretinoin, valcyclovir, acyclovir, delavirdin, famciclovir, lamivudine, zalcitabine, osteltamivir, abacavir, prilosec,

Vitamins useful in the practice of the present invention include, but are not limited to, A, C, E, and K.

Flavors and fragrances useful in the practice of the present invention include, but are not limited to, vanillin, methyl salicylate, thymol, ethyl vanillin, acesulfame, and saccharin.

Water treatment and detergent additive compounds useful in the practice of the present invention include, but are not limited to, polymers and copolymers of acrylic acid or methacrylic acid with other polymerizable monomers such as acrylamidomethyl propane sulfonic acid, ethyl acrylate, acrylamide and alkyl derivatives of acrylamide, allyl hydroxypropylether sulfonic acid, and their salts used as dispersants and scale inhibitors, phosphonate compounds such as 1-hydroxyethilidene-1,1-diphosphonic acid, aminotris(phosphonic acid), phosphonobutane tricarboxylic acid, and hydroxyphosphonoacetic acid, used as scale inhibitors or corrosion inhibitors, aminotris(acetic acid) and ethylene diamine tetraacetic, used as chelants, quaternary nitrogen compounds such as alkyldimethylbenzylammonium chloride, used as biocides.

Agricultural compounds useful in the practice of the present invention include, but are not limited to, ferbam, fosetyl-aluminum, glufosinate, glyphosate, pesticides that contain carboxyl groups, such as (2,4-dichlorophenoxy)acetic acid, 4-chloro-2-methylphenoxybutyric acid, , 4-chloro-2-methylphenoxyacetic acid, herbicides such as diphenylethers and the dithiocarbamates, and pesticides that contain amino groups.

The active ingredient component of the composition may be present in any amount which is sufficient to elicit a beneficial effect.

Preferably the range of the ratio of unloaded resin to active ingredient of the present invention is 1000:1 to 0.01:1 by weight. More preferably the range of the ratio of unloaded resin to active ingredient of the present invention is 100:1 to 0.1:1 by weight. Most preferably the range of the ratio of unloaded resin to active ingredient of the present invention is 20:1 to 0.1:1 by weight.

The preferred temperature range for the practice of the present invention is -10°C to 150°C, the more preferred range is 0°C to 100°C, the even more preferred range is 5°C to 60°C, and the most preferred range is 5°C to 50°C.

While not wishing to be bound by theory, Applicants propose that an absorption/ release process is occurring. For example, when the present invention is used in the pharmaceutical arts, a mixture of an unloaded resin and an ionizable active ingredient are exposed to the release medium, such as intestinal fluid, the ionizable active ingredient starts to dissolve in said fluid. A part of this dissolved ionizable active ingredient is absorbed by the unloaded resin because the system attempts to achieve equilibrium. The ionizable active ingredient that is absorbed by the unloaded resin is not, therefore, absorbed by the body at this stage. The dissolved part that is not absorbed by the unloaded resin is absorbed by the body. This state, where ionizable active ingredient is dissolving and being absorbed by both the resin, which is now partially loaded, and the body will continue until the amount of ionizable active ingredient on said partially loaded resin is in equilibrium with the ionizable active ingredient in solution. After this point further decrease in the concentration of the ionizable active ingredient by absorption into the body will result in the release of the ionizable active ingredient that has previously been absorbed by the resin, and the release rate profile will be the same as if pre-made resinate had been used. Hence, the benefit of a resinate is achieved without the cost of resinate manufacture.

The unloaded resin must be chosen such that under conditions of use a significant amount of the active ingredient in solution is absorbed by said resin. Current understanding in the industry does not permit prediction *a priori* of the types of unloaded resin/ionizable active ingredient combinations that fulfill this requirement. The suitable resin/ionizable active ingredient combination can be determined by techniques known to those skilled in the art. For example, one can measure the uptake of the ionizable active ingredient by the resin from a solution of the active ingredient in the release medium using a simple spectrophotometric absorption analysis as described herein. The spectrophotometric data can serve as a guide to selecting appropriate resin/active ionizable ingredient combinations.

The final dosage form can be any of the many variations known in the art, provided that they do not result in transfer of the active ingredient onto the unloaded resin during storage and prior to use. These can include, but are not limited to, tablets, powders, pills, syrups, hard capsules and soft capsules.

The unloaded resin and the ionizable active ingredient do not have to be mixed in the formulation, provided that the formulations allows the ionizable active ingredient and the unloaded resin to be present in the same body of fluid at the same time when in use. In those cases where the unloaded resin and ionizable active ingredient are mixed prior to use, any of the known methods for preparing mixtures of solids can be used in the practice of this invention. See, Remington's Pharmaceutical Sciences, 16^{th} Edition, 1980, Chapter 88.

The invention is not restricted to the use of only one unloaded resin. Multiple unloaded resins can also be used as needed to produce the desired release rate profile.

The invention is not restricted to the use of only one active ingredient. Multiple active ingredients can be used to produce the desired beneficial effect.

The rate of release of ionizable active ingredients from unloaded resins or absorption of ionizable active ingredients onto unloaded and partially loaded resins may depend on multiple factors which are well known in the industry. These include, but are not limited to, degree of cross-linking of the unloaded resin, the particles size of the unloaded resin, the pK of the functional groups of the unloaded resin, the solubility of the active ingredient in the release medium, the ionic strength and pH of the release medium, the pK of the active ingredient, the molecular weight of the active ingredient, and the temperature. Coating the unloaded resin with a permeable membrane can also change the rate of release or absorption. Coating the unloaded resin with non-permeable membranes can change the conditions under which the release and absorption takes place depending on the conditions under which the membrane dissolves.

By balancing the variables that control the rate of absorption of the active ingredient by the unloaded resin and subsequent rate of release, the shape of the release rate curve can be adjusted to be equivalent to that obtained using pre-made resinate.

The practice and utility of the present invention is described hereinbelow.

### Example 1 - Preparation of Diclofenac/Cholestyramine resinate

For the purposes of comparison with the present invention, a sample of diclofenac/cholestyramine resinate was prepared using the method described in US Patent 4,510,128, Example 1(a). 5g of diclofenac sodium and 5g of conditioned cholestyramine resin were used and produced 8.24g of resinate. Cholestyramine is a styrenic strongly basic anion exchange resin with a quaternary amine functionality having a weight capacity of 4.0 to 4.5 meq/g.

### Example 2 - Release test on dicofenac/cholestyramine resinate

Equipment was set up as follows: A 50 ml continuous, stirred, filtration cell, such as the Amicon stirred ultrafiltration cell model 8050, available from Millipore Corporation, was equipped with a peristaltic pump to feed fluid into the cell at a rate in the range 3-10ml/min. The filtrate from the cell was passed into a 1cm path length flow-through quartz uv cell. The uv cell is situated in a suitable uv spectrophotometer, such as the Genesys 2, UV Spectrophotemer available from Spectronic Instruments. The filtration cell is fitted with a 3 micron filter to retain the resin particles. 50ml of simulated intestinal fluid was added to the filtration cell, and then simulated intestinal fluid was fed into it via the pump at a flow rate of 6.2+/-0.2ml/min. This was continued until the absorbance at 276nm as measured in the uv cell was constant. 92.8mg of the resinate, equivalent to 50mg of diclofenac sodium prepared as in Example 1 was then added to the filtration cell. The uv absorbance at 276nm of the effluent from the cell was recorded through the duration of the test. The diclofenac sodium concentration was calculated using a suitably determined calibration curve. The results of Example 2 are shown in Table 1 expressed as the instantaneous concentration in the effluent. The example illustrates the release rate profile from a pre-made resinate that is typical of the current art.

### Example 3 - Release test using a combination of diclofenac sodium and unloaded cholestyramine, ratio 1:1

The procedure of Example 1 was repeated except that 51.0mg of diclofenac and 50.1mg of unloaded, unconditioned, cholestyramine USP that had been screened to remove particles >37 microns were used. The results of Example 3 are shown in Table 1 expressed as the instantaneous concentration in the effluent. In Example 3 the amount of diclofenac and unloaded resin is the same as in Example 1 resinate (ratio 1:1). However, Example 3 gives a faster release rate.

### Example 4 - Release test using a combination of diclofenac sodium and unloaded cholestyramine, ratio 1:1.5

The procedure of Example 1 was repeated except that 51.7mg of diclofenac and 75.9mg of unloaded, unconditioned, cholestyramine USP that had been screened to remove particles >37 microns were used. The results of Example 4 are shown in Table 1 expressed as the instantaneous concentration in the effluent. In Example 4 the amount of diclofenac is the same as in Example 2, but the amount of resin is different. The data clearly shows that the release rate profile for Example 4 is essentially identical to that of Example 2. This demonstrates that in the present invention the release rate profile can be adjusted to achieve a profile equivalent to that obtained from a pre-made resinate.

### Spectrophotometric Absorption Test Method

It is important that the drug be absorbed by the resin. The following test method is useful in determining drug absorption by the resin. In this illustration of the test method, indomethacin and cholestyramine are used. Equipment was set up as described in Example 1, except that the effluent from the uv cell was directed back to the inlet of the pump to produce a circulation flow. 55ml of a 98mg/l solution of indomethacin in simulated intestinal fluid, prepared as described below, was charged to the filtration cell and the pump and stirrer started. Flow rate was approximately 6 ml/min. Absorbance readings were taken on the uv spectrophotometer at a wavelength of 318nm until a steady baseline was obtained. 43mg of Cholestyramine USP that had been screened to remove particles <75 microns was then added to the cell. Absorbance readings were then taken at frequent intervals to observe the uptake of indomethacin. The absorbance readings were used to calculate the indomethacin concentration from a suitably determined calibration curve and to calculate the total amount of indomethacin absorbed by the resin. In the case of the indomethacin, 92% was absorbed within 5 hours.

**Preparation of simulated intestinal fluid:** a solution of 6.8g/l potassium dihydrogen phosphate in deionized water was prepared and sufficient 0.2mol/l sodium hydroxide solution was added to achieve a pH of 7.5.

**Table 1**

| **Instantaneous concentration of diclofenac sodium in the release medium (mg/l)** | | | |
|---|---|---|---|
| **Time (min)** | **Example 2 (resinate)** | **Example 3 (present invention)** | **Example 4 (present invention)** |
| 10 | 62.1 | 110.9 | 59.9 |
| 20 | 50.2 | 76.5 | 50.3 |
| 30 | 42.2 | 55.1 | 42.0 |
| 60 | 28.5 | 28.4 | 27.4 |
| 120 | 16.5 | 13.5 | 15.8 |
| 240 | 8.4 | 5.3 | 8.4 |
| 480 | 3.3 | 1.6 | 3.7 |
| 720 | 1.5 | 0.6 | 2.1 |
| 960 | 0.7 | 0.2 | 0.9 |
| 1200 | 0.2 | 0.0 | 0.2 |
| 1440 | 0.0 | 0.0 | 0.0 |

Table 1 illustrates the amount of diclofenac sodium in the release medium over time from a resinate and from dosage forms prepared according to the present invention.

## Claims

1. A dosage form comprising:
a. an ionizable active ingredient;
b. an unloaded ion exchange resin.

2. A dosage form comprising:
a an ionizable active ingredient;
b. an unloaded ion exchange resin;
further provided that the release rate and absorption rate of said ionizable active ingredient from said dosage form into a release medium can be modified by changing the variables, selected from the group consisting of, degree of cross-linking of the unloaded ion exchange resin, the particle size of the unloaded ion exchange resin, the pK of the functional groups of the unloaded resin, the solubility of the ionizable active ingredient in the release medium, the ionic strength and pH of the release medium, the pK of the ionizable active ingredient, the molecular weight of the ionizable active ingredient, temperature, and coating the unloaded ion exchange resin with a permeable membrane.

3. A dosage form comprising:
a. an ionizable active ingredient;
b. an unloaded ion exchange resin;
further provided that the site at which release and absorption take place can be modified by coating the unloaded resin and/or active ingredient with a non-permeable membrane that is dissolved only at the site where absorption and release are desired.

4. A dosage form according to Claim 1, wherein said ionizable active ingredient is selected from the group consisting of diclofenac sodium, indomethacin, and pesticides containing carboxyl groups, and said unloaded ion exchange resin is selected from the group consisting of styrenic strongly basic anion exchange resins with a quaternary amine functionality having a weight capacity of 0.1 to 6 meq/g, and styrenic weakly basic anion exchange resins with a tertiary amine functionality having a weight capacity of 0.1 to 8.5 meq/g, acrylic or methacrylic strongly basic anion exchange resins with a quaternary amine functionality having a weight capacity of 0.1 to 8 meq/g, and acylic or methacrylic weakly basic anion exchange resins with tertiary amine functionality having a weight capacity of 0.1 to 12 meq/g, and allylic and vinylic weakly basic anion exchange resins with primary, secondary, or tertiary amine functionalities having a weight capacity of 0.1 to 24 meq/g.

5. A dosage form according to Claim 1, wherein said ionizable active ingredient is selected from the group consisting of paroxetine, and quaternary nitrogen compounds, and said unloaded ion exchange resin is selected from the group consisting of styrenic strongly acidic cation exchange resins with a sulfonic acid functionality having a weight capacity of 0.1 to 8 meq/g; and styrenic weakly acidic cation exchange resins with a phenolic acid functionality having a weight capacity of 0.1 to 8.5 meq/g; and acrylic or methacrylic weakly acidic cation exchange resins with a carboxylic or phenolic acid functionality with a weight capacity of 0.1 to 14 meq/g.
